# EUROPEAN PATENT APPLICATION

(11) **EP 1 698 693 A1**
(43) Date of publication of application: **06.09.2006**
(21) Application number: 04773573.3
(22) Date of filing: 27.09.2004
(51) Int. Cl.: C12N 15/00, C07K 14/435, C12N 1/15, C12N 1/19, C12N 1/21, C12N 5/00, A01H 5/00, A01K 67/027, A01K 67/033

(54) **ROYAL JELLY PEPTIDE AND COMPOSITION CONTAINING THE SAME**

(30) Priority: 29.09.2003 JP 2003338665
(71) Applicant: Japan Science and Technology Agency, Kawaguchi-shi, Saitama 332-0012 (JP)
(72) Inventor: SUZUKI, Koichi, Morioka-shi, Iwate 020-0834 (JP); SAKAI, Masato, 102, Kakuno Haitsu, 79, Abuta-gun, Hokkaido 049-5605 (JP)
(74) Representative: Owen, Deborah Jane
(86) International application number: PCT/JP2004/014544
(87) International publication number: WO 2005/030951

(57) **Abstract**

A royal jelly peptide which has a function as a queen bee' s attraction cofactor and can enhance the biological defense system of an animal (an immunopotentiating effect); and a composition containing the same.

## Description

### Technical Field

The invention of this application relates to a royal jelly peptide and a composition containing the same. More particularly, the invention of this application relates to a royal jelly peptide which has a function as a cofactor for induction to queen bees, and can implement the improvement of the animal biological defense system (immunity activating effect), and a composition containing the same.

### Background Art

As honeybee products, honey and royal jelly are well known. Particularly, as distinct from honey mostly made of carbohydrates, royal jelly contains various vitamins, minerals, and the like, including proteins, carbohydrates, and lipids which are three major nutrients, in a balanced amounts, and richly. The royal jelly is regarded as having not only the effect of induction to queen bees, but also various effects such as being effective for recovery from fatigue, allergy, infectious disease, cancer, and the like for animals including a human.

For this reason, it is considered that royal jelly is artificially fed to honeybee larvae to induce differentiation of the larvae to queen bees. This enables the indoor artificial breeding of queen bees, so that systematic honeybee production can be expected. However, in actuality, such indoor artificial breeding of queen bees is difficult.

Whereas, on the other hand, for a human, royal jelly has received attention in a large number of food fields and the like, and hence it has been used as one of health foods. However, in actuality, the foregoing wide-ranging effects of royal jelly cannot be said to have been proved scientifically and clearly.

Under such circumstances, in order to solve such problems, there have been increasing studies on royal jelly. First, there is proposed a method for inducing differentiation to queen bees in order to implement the indoor artificial breeding of queen bees (JP-A-5-076253). As described above, it is known that the effects of royal jelly on honeybees include a function of inducing differentiation of honeybee larvae to queen bees. By extracting and purifying a proteinaceous substance having this function, and feeding it to honeybee larvae, it is possible to induce differentiation of the larvae to queen bees with efficiency. This proposed method enables the indoor artificial breeding of queen bees of honeybees, so that systematic honeybee production can be carried out.

On the other hand, secondly, there have been made a large number of studies for elucidating the wide-ranging effects possessed by royal jelly. For example, the protein, the peptide, and the like which are the royal jellyforming components are identified, and the functional analysis of the respective components are carried out. For example, major proteins 1 to 5 (MJR 1 to 5) have been reported, and it is disclosed that particularly MJR 3 promotes the immune response (Okamoto, I., et al., Life Sciences, 73, p2029-2045, 2003). Whereas, there is another report suggesting the possibility that apisin promotes the growth of a cell, and other possibilities (Masami Yonekura, MITSUBACHI KAGAKU, vol. 19, No. 1, pages 15 to 22, 1998, and Yonekura, M., New Food Ind., 41 (1) p 1 to 8, 1999), and there are a still other report that royalisin has an antibacterial property (Fujiwara, S., et al., J. Biol. Chem., 265 (19), p11333-7, 1990), and other reports. Further, in recent years, apisimin has been identified as one of the royal jelly-forming components (Bilikova, K., et al., FEBS Letters, 528, p125-129, 2002).

However, in the case of the method proposed in the Patent Document 1 exemplified as the method for inducing differentiation to queen bees, for extraction of the differentiation inducing factor used in this proposal from royal jelly, unfavorably, a complicated procedure with an organic solvent, ultrafiltration, or the like is required to be carried out.

Whereas, the functional substances contained in royal jelly identified at present are only the MJR 1 to 5, apisin, royalisin, and apisimin. In actuality, the functionalities thereof have not been clarified in details. Particularly, as for the recently found apisimin, what functions such as physiological functions it has, the functionality has not been elucidated at all.

Thus, the invention of this application has been made in view of the foregoing circumstances. It is an object to provide a royal jelly peptide which solves the conventional problems, and has a function as a queen bee differentiation inducing cofactor, and can implement the improvement of the animal biological defense system (immunity activating effect), and a composition containing the same.

### Disclosure of the Invention

The invention of this application provides the following inventions (1) to (11) as the means for solving the foregoing problems:
(1) A royal jelly peptide characterized by having at least any function activity of a function as a queen bee differentiation inducing cofactor, and a function as an animal immunity activating factor.
(2) An expression vector including an expression cassette having a gene sequence coding for the royal jelly peptide according to the item (1).
(3) A transgenic cell transformed by the expression vector according to the item (2).
(4) A royal jelly peptide produced by the transgenic cell according to the item (3).
(5) A composition containing the royal jelly peptide according to the item (1) or (4) as an effective component, characterized by having at least any function of a function of improving the auxiliary effect of inducing differentiation of honeybees to queen bees, and a function of improving the animal immunity activating effect.
(6) A method for inducing honeybees to queen bees characterized by administering the composition according to the item (5) to honeybee larvae, thereby promoting the growth of the larvae, and inducing differentiation to queen bees.
(7) A method for activating the animal immunity, characterized by administering the composition according to the item (5) to an animal, and thereby improving the animal immunity activating effect.
(8) Transgenic organisms being a non-human organism obtained by ontogenesis of any of an early embryo, a fertilized egg, and an embryonic stem cell of a non-human organism into which a gene sequence coding for the royal jelly peptide according to the item (1) has been transferred, and a progeny organism thereof, characterized by having the gene in a somatic cell chromosome, and expressing the royal jelly peptide.
(9) The non-human organism according to the item (8), characterized in that the non-human organism is any of a plant or an animal.
(10) The non-human organism according to the item (9), characterized in that the animal is an insecta.
(11) The non-human organism according to the item (10), characterized in that the insecta is a silkworm.

Then, the foregoing inventions of this application can provide the following effects.

With the royal jelly peptide of the invention according to the item (1), it is possible to artificially aid the induction of honeybee larvae to queen bees, and it is possible to activate the animal immunity.

With the expression vector of the invention according to the item (2), the expression vector is constructed to have a gene sequence coding for the royal jelly peptide, thereby resulting in improvements of the storability and the ease of handling.

With the transgenic cell of the invention according to the item (3), it is possible to express the royal jelly peptide with stability and simplicity.

With the royal jelly peptide of the invention according to the item (4), it is possible to obtain the same effects as with the invention according to the item (1). Further, the royal jelly peptide can be produced from the transgenic cell having the same origin as that of the animal targeted for administration, and hence it can repress the immunogenicity.

With the composition of the invention according to the item (5), in addition to the effects of the invention according to the item (1) or (4), by mixing with various pharmacological components or the like, or performing processing of sugarcoating, encapsulation body, or the like, or other process, it is possible to facilitate ingestion of the composition containing the royal jelly peptide as an effective component.

With the method for induction to queen bees of the invention according to the item (6), the indoor artificial breeding of queen bees becomes possible, and this can implement the systematic honeybee production.

With the method for activating the animal immunity of the invention according to the item (7), it becomes possible to improve the function of the immunity defense system of animals including a human subjected to administration.

With the transgenic organism of the invention according to the item (8), it is possible to obtain an organism more activated and enhanced in a large number of physiological functions such as high productivity, disease resistance, heat resistance, and cold resistance.

With the non-human organism of the invention according to the item (9), it is possible to obtain the same effects as with the invention according to the item (8), and it becomes possible to obtain higher effects.

With the non-human organism of the invention according to the item (10), it is possible to obtain the same effects as with the inventions according to the item (8) and (9), and it becomes possible to obtain still higher effects.

With the non-human organism of the invention according to the item (11), a silkworm can be obtained as a transgenic organism having the effect of royal jelly peptide more effectively, because abundant findings have been made on the breeding method and the like.

### Brief Description of the Drawings

FIG. 1 is a diagram showing the outline of a protocol for extracting a royal jelly peptide from a royal jelly powder;
FIG. 2 is a diagram showing the high performance liquid chromatography elution pattern of the royal jelly peptide of the invention of this application, and the analysis by SDS-PAGE at the peak (arrow site);
FIG. 3 is a diagram showing the amino acid at the N terminal of the royal jelly peptide of the invention of this application;
FIG. 4 is a diagram showing the base sequence and the amino acid of the royal jelly peptide of the invention of this application;
FIG. 5 is a photographic diagram showing the result of analysis of the genome DNA of Western honeybees by the Southern blotting process;
FIG. 6 is a photographic diagram showing the result of analysis of the total RNA of working honeybees by the Northern blotting process;
FIG. 7 is a diagram showing the base sequences of fragments 1 to 8 used for the construction of the royal jelly peptide of the invention of this application;
FIG. 8 is a diagram showing the high performance liquid chromatography elution pattern of a recombinant royal jelly peptide, and the analysis result by SDS-PAGE at the peak (horizontal bar);
FIG. 9 is a diagram showing the effect of the recombinant royal jelly peptide exerted on the growth of the lymphocyte cell derived from a mouse spleen; and
FIG. 10 is a photographic diagram showing that the organ for synthesizing the royal jelly peptide is the hypopharyngeal gland by using an anti royal jelly peptide antibody.

### Best Mode for Carrying Out the Invention

The invention of this application has the features as described above, and the embodiments thereof will be described below.

The royal jelly peptide of this application is one of the royal jelly components produced by honeybees, and reported as "apisimin" (Bilikova, K., et al., FEBS Letters, 528, p125 to 129, 2002). The sequence of the gene coding for this is also disclosed (Gen Bank Accession No. AAQ16586 and Gen Bank Accession No. AY340960). However, no elucidation on the function has been made. Thus, the invention of this application has been made based on the results of the close study of the inventors. Namely, the royal jelly peptide of the invention of this application has been provided based on the elucidation of the following fact. The royal jelly peptide has a function as a queen bee differentiation inducing cofactor which is administered to honeybee larvae, and thereby induces the differentiation of the larvae to queen bees. Further, it also has a function as an immunity activating factor which is administered to animals including a human, and thereby improves the immunity of the animals. As a result of this, it is also possible to artificially induce honeybee larvae to develop into queen bees, and it is also possible to improve the immunity of animals with simplicity, resulting in enhancements of resistance against and resilience from infectious diseases and the like.

As in the related art, the royal jelly peptide of the invention of this application can also be produced from royal jelly with known extraction methods and purification methods such as extraction using an organic solvent and ultrafiltration as in the related art. However, the royal jelly peptide of the invention of this application can be produced by using genetic engineering methods, and hence it is preferred. Specifically, for example, by transferring an expression vector including an expression cassette having a gene sequence coding for the royal jelly peptide (e.g., Gen Bank Accession No. AAQ16586 and Gen Bank Accession No. AY340960) into an Escherichia coli, a yeast, a plant cell, an animal cell, or the like according to a known method such as an electroporation method, a calcium phosphate method, or a microinjection method, it is possible to achieve the production on a mass scale and with simplicity. Further, the royal jelly peptide collected from the cell body / cell of the transformed Escherichia coli, yeast, plant cell, animal cell, or the like can be purified with high purity and high yield. Further, inclusion of the sequence coding for 6 x histidine-tag (His), FLAG, β-galactosidase, glutathione-S-transferase (GST), c-myc, maltose binding protein (MBP), or the like as a tag sequence facilitates the purification.

The "expression vector" in the invention of this application can be produced by inserting and ligating the expression cassette having a gene sequence coding for the royal jelly peptide into the base vector. Therefore, the expression cassette preferably has a restriction enzyme sequence corresponding to a given cloning site of the base a vector. The "base vectors" include, for example, various base vectors such as a vector for an animal cell, a vector for an insect cell, a vector for a yeast, a vector for Escherichia coli, and a shuttle vector for a plurality of species including a yeast / Escherichia coli, and the like. These base vectors may be appropriately selected according to the host cell and the intended purpose. Alternatively, an existing vector DNA for expressing a foreign protein in an appropriate host cell can be partially altered to be used. For example, when a microorganism such as Escherichia coli is used as a host cell, pUC series, pBlue script II series, and pET series systems and the like, having origins, promoters, terminators can be used.

The bacteria and cells (receptors) for use in transferring the expression vectors have no particular restriction so long as they can express the royal jelly peptide with stability. For example, Escherichia coli, yeasts, various plant cells, various animal cells, and the like can be used. Thus, the type of the cell targeted for transformation can be arbitrarily selected, and used. Therefore, by appropriately selecting the origin and the type of the cell to be transformed in accordance with the origin of animals including a human targeted for administration of the royal jelly peptide, it is possible to repress the immunogenicity.

Further, the invention of this application provides a composition containing a royal jelly peptide having the foregoing features as an effective component. Namely, this composition has a function of inducing differentiation of honeybee larvae to queen bees, and has a function of improving the immunity of animals including a human. This composition can be mixed with pharmacological components, food components, or the like, and processed, resulting in formulation or functional food which is easy to administer or to ingest. Any substances can be mixed and processed without a particular restriction so long as they are substances useful for a living body such as various vitamins and various minerals which are one types of food components, and capable of keeping the effects of the royal jelly peptide of this application.

Incidentally, the "pharmacological components" are various carriers to be generally utilized for pharmaceutical drug production. The carrier can be appropriately selected according to the type of the pharmaceutical drug, the administration form such as injection or oral administration, or the like. For example, pharmaceutical drugs in an oral liquid form such as a suspension and a syrup can be produced by using water, saccharides such as sucrose, glycols such as polyethylene glycol, oils such as sesame oil and soybean oil, and other than these, an antiseptic, various flavors such as peppermint, and the like. Whereas, a powder, a pill, a capsule, and a tablet can be prepared in a formulation by using an excipient such as lactose, glucose, or sucrose, a disintegrator such as starch, a lubricant such as magnesium stearate, various binders, a surfactant, a plasticizer, and the like.

By allowing the composition to be ingested by honeybee larvae as food or the like, it is possible to artificially induce differentiation to queen bees. As a result, the indoor artificial breeding of queen bees which has been difficult in the related art becomes possible. This enables the systematic production of honeybees, which can implement efficient acquisition of royal jelly, honey, and the like. Whereas, by allowing the composition to be administered to, or to be ingested by animals including a human, it is possible to activate or enhance the immunity. As a result, the resistance against or the resilience from various diseases is enhanced, so that health can be expected to be kept.

Further, the invention of this application provides non-human transgenic organisms in which a gene sequence coding for a royal jelly peptide has been transferred. The transgenic organism is a non-human organism obtained by ontogenesis of any of the early embryo, fertilized egg, and embryonic stem cell (ES cell) of an organism, and a progeny organism thereof, and have features of having a gene sequence coding for the royal jelly peptide in the somatic cell chromosome, and expressing the royal jelly peptide. The transgenic organism can be produced by, for example, a known transgenic animal production process (e.g., Gordon, JW. et al., Proc. Natl. Acad. Sci. USA, 77, p7380 to 7384, 1980) for an animal. Namely, an objective transgenic animal can be produced in the following manner. A functional gene (the gene coding for the royal jelly peptide in this application) is transferred to totipotent cells such as early embryos, fertilized eggs, or embryonic stem cells, and the cells are developed into individuals. Thus, the individual in which a foreign functional gene has been incorporated in the genome of the somatic cell is screened.

The "transfer of a functional gene" is the transfer of a foreign functional gene into the chromosome genome by the transgenic animal production process. Whereas, by using a target gene recombination process which is known (gene targeting process) (Capecchi, MR., et al., Science, 244, p1288 to 1292, 1989), it is also possible to transfer a foreign functional gene by substitution with an endogenous functional gene. Incidentally, the foreign functional gene is the gene inherently not present in the chromosome genome of the organism, and a gene derived from another organism, a synthetic gene produced by PCR or the like, or other gene.

Incidentally, the ES cells have been confirmed for a mouse, a rat, a rabbit, a monkey, and the like. Whereas, for a pig, or the like, the EG (embryonic germ) cell has been confirmed, and hence the animals can be taken as the target animal species.

For a plant, by using a process utilizing transfer of t-DNA in Ti plasmid into a plant cell and chromosome incorporation occurring upon infection of Agrobacterium tumefaciens (particularly, preferable for dicotyledon), or a process of direct transfer of DNA into a protoplast with an electroporation process or the like (particularly, preferable for monocotyledon such as rice), or the like, it is possible to transfer a functional gene (a gene coding for the royal jelly peptide).

The foregoing transgenic animals or plants can provide organisms enhanced in various functions such as disease resistance, weather resistance, cold resistance, and heat resistance. Further, for plants which are crops such as rice, it is possible to expect the improvement of the productivity, which can be put to use for domestic animals and field crops.

Still further, the animal in the transgenic animal is preferably insecta. The insecta is preferably a silkworm from the viewpoints of the high efficiency and the ease of handling because abundant findings on the breeding method and the like have been accumulated therefor.

Below, Examples will be shown, and the invention of this application will be described in more details. It is understood that the invention of this application is not limited to the following Examples.

### Examples

### Example 1: Extraction of royal jelly peptide

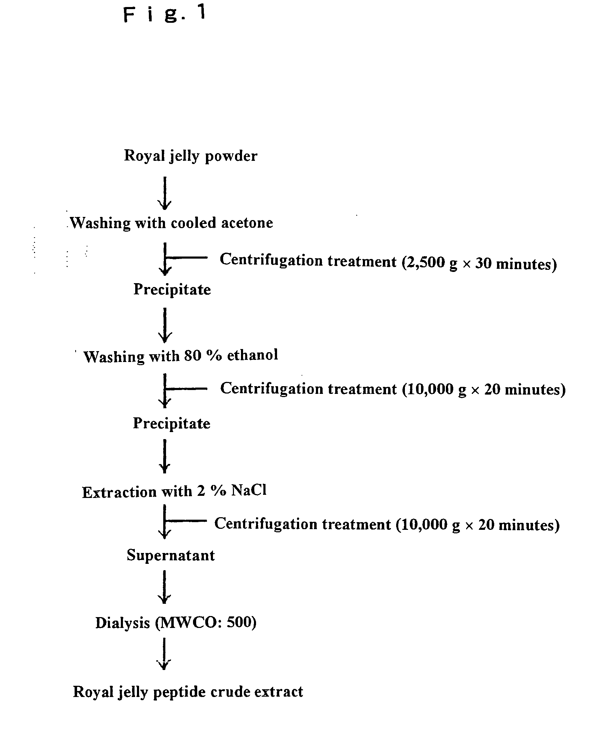

From a royal jelly in a dry powder form, a crude fraction containing the objective royal jelly peptide was extracted by the extraction means shown in FIG. 1. Namely, a royal jelly in a dry powder form was washed with cooled acetone, and the solution was subjected to a centrifugal treatment at 2500 x g for 30 minutes. The precipitate was collected, and washed with 80 % ethanol, and subjected to a centrifugal treatment at 10000 x g for 20 minutes. Again, the precipitate was collected, and an extraction operation was carried out using 2 % NaCl. Then, a centrifugal treatment was carried out at 10000 x g for 20 minutes, and a crystallization operation (MWCO: 500) was carried out, thereby to obtain a crude fraction containing the objective royal jelly peptide.

### Example 2: Isolation of royal jelly peptide

The crude fraction of apisimin obtained in Example 1 was subjected to isolation by high performance liquid chromatography with the peak eluting using an acetonitrile concentration of 96 % as a single royal jelly peptide.

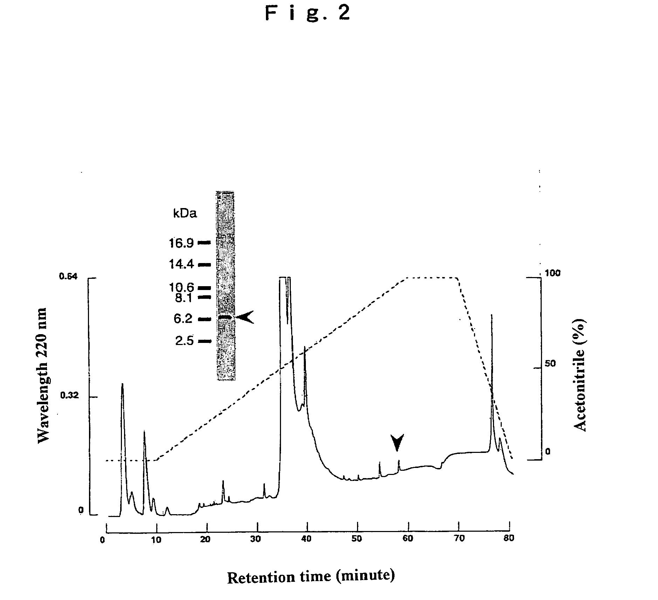

FIG. 2 shows the elution pattern in the high performance liquid chromatography of a royal jelly peptide. As shown in FIG. 2, for the crude fraction containing a royal jelly peptide, the arrow site (peak) indicated with reversed phase high performance liquid chromatography by TSK-gel ODS-80Ts was analyzed by Tricine SDS-PAGE. As a result, it could be isolated as a single royal jelly peptide (6.2 kDa).

### Example 3: Determination of partial amino acid sequence of royal jelly peptide

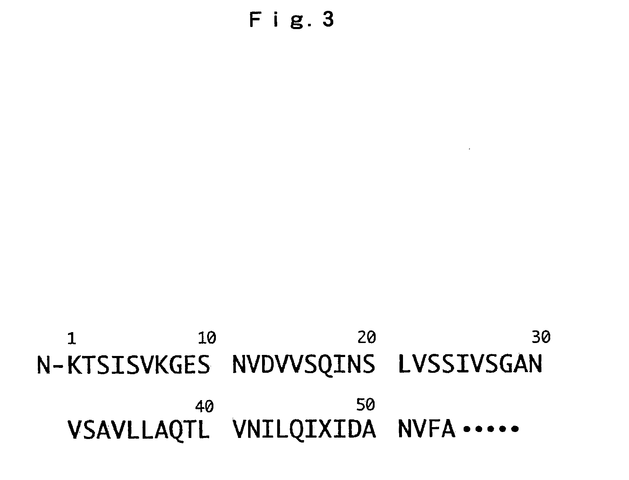

The first structure of the royal jelly peptide isolated in Example 2 was analyzed by means of a fully automated protein first structure analyzing system (protein sequencer) (G-1000A, manufactured by Hewlett-Packard). Thus, the amino acid sequence from the N-terminal side was determined with a known technology, Edman degradation method. As a result, as shown in FIG. 3, the amino acid sequence including 54 amino acid residues in which the 47th amino acid from the N-terminal side is unknown could be determined (sequence No. 14 and sequence No. 15).

Incidentally, the measurement of the molecular mass of the royal jelly peptide was also carried out (not shown). For this measurement, MALDI-TOF MS system Voyager RP (trade name) (manufactured by PerSeptic Biosystem Co.) which is a mass spectrometer was used. The measurement was carried out in the following manner. α-cyano-4-hydroxycinnamic acid (α-CHCA) saturated in 50 % acetonitrile was used as a cationizing matrix. Calibration Mixture 2 (ACTH (7-38 clip), insulin (bovine)) which is a molecular weight marker, and a peptide dissolved in 0.1 % TFA were mixed on a plate, and air-dried, followed by laser irradiation, thereby to obtain the molecular mass.

### Example 4: Determination of the full length of cDNA coding for a royal jelly peptide

### <1> Breeding method of honeybee

Poly A+RNA was extracted from the hypopharyngeal glands (organ for producing royal jelly protein) of Western honeybees (Apis mellifela) (provided from Fujiwara beehouse, Morioka city) by a known process (e.g., Sambrook, J., et al., Molecular Cloning, Cold Spring Harbor Laboratory Press, 1989).

Incidentally, the Western honeybees were bred outdoors using hives (movable hives) for Western honeybees in the botanical garden affiliated to the faculty of agriculture, Iwate University. In the autumn period in which nectar is in short, food obtained by mixing edible sugar and water (in a ratio of 1:1) was given thereto. Further, as a countermeasure against Varrora jacobsoni which is an enemy, Apistan (manufactured by Mitsubishi Chemical Corp.) which is an anti-mite agent was used. In the winter period, the hives were surrounded by Styrofoam in order to keep the colonies. Further, the thoraces of working bees immediately after eclosion were marked with an enamel paint. By using this as the indicator, 6- to 10-day old working bees (nurse bees) whose hypopharyngeal glands had been under active development were collected and used.

### <2> Excision of hypopharyngeal glands

Working bees were cold anesthetized on ice to collect the heads, and the hypopharyngeal glands were excised from the heads in a physiological salt solution for cooling the bees (0.02 % (w/v) KCI, 2.02 % (w/v) CaCl₂, 0.4 % (w/v) saccharose, and 0.9 % (w/v) NaCl).

### <3> Control of Total RNA

### (a) Extraction of Total RNA

For extraction of total RNA, Quick Prep (trade name) Total RNA Extraction Kit (manufactured by Amersham Pharmacia Biotch Co.) was used. According to the attached protocol, Total RNA was extracted from 53 mg of the hypopharyngeal glands. After ethanol precipitation, it was dissolved in 200 µl of sterilized water. Then, a portion thereof was measured for the wavelength A₂₅₀ by means of a spectrophotometer, to calculate the concentration.

### (b) Isolation of poly A⁺ RNA

From 500 µg of the obtained Total RNA, poly A⁺ RNA was isolated according to the attached protocol by means of Oligotex-dt30 <Super> (manufactured by Takara Shuzo Co., Ltd.). The measurement of the concentration of the poly A⁺ RNA was carried out in the same manner as with the item (a).

### <4> Cloning of royal jelly peptide using Rapid Amplification of cDNA Ends (RACE)

### (a) Synthesis of cDNA

By means of Marathon (trade name) cDNA amplification Kit (manufactured by CLONTECH Co.), the RACE process was carried out according to the attached protocol. 1 µg of poly A⁺ RNA obtained in the items (a) and (b) of the above <3> was allowed to react, together with Marathon cDNA Synthesis Primer (TTCTAGAATCAGAGGAAGAT₍₃₀₎ N_{_1}N) at 70 °C for 10 minutes in a sterilized water, and then, quenched on ice. The reaction solution was allowed to react at 72 °C for 1 hour in 10 µl of First-strand Buffer containing 10 nmol dNTP, and 100 units of MMLV Reverse Transcriptase (Superscript (trade name) II, manufactured by Gibco BRL CO.), to synthesize a single stranded cDNA.

Further, the reaction solution was allowed to react at 16 °C for 1.5 hours in 80 µl of Second-strand Buffer (100 mM KCI, 10 mM Ammonium sulfate, 5 mM MgCl2, 0.15 mM β-NAD, 20 mM Tris (pH 7.5), 0.05 mg/ml BSA) containing 10 nmol dNTP and Second-strand Enzyme Cocktail (24 units of E. coli DNA Polymerase I, 0.8 units of E. coli DNA ligase, 1 unit of E. coli RNaseH). After the reaction, 10 units of T4 DNA Polymerase was added thereto, and the reaction was allowed to proceed at 16 °C for 45 minutes to synthesize a double stranded cDNA.

The double stranded cDNA was extracted with phenol / chloroform / isoamyl alcohol (25 : 24 : 1), and further extracted with chloroform / isoamyl alcohol (24 : 1). In the extracted solution, cDNA was precipitated with 1/2 volume of 4 M sodium acetate and 2.5 volume of ethanol, and washed with 80 % ethanol.

### (b) Adaptor ligation

The precipitate of double stranded cDNA was dissolved in sterilized water, and was allowed to react at 16 °C overnight in 10 µl of DNA Ligation Buffer (50 mM Tris-HCl (pH 7.8), 10 mM MgCl₂, 1 mM DTT, 1 mM ATP, 5 % Polyethylene Glycol (MW: 8000)) containing Marathon cDNA and 1 unit of T4 DNA Ligase. The adaptor-ligated cDNA was diluted to 50 times with a TE buffer (40 mM Tris-HCl (pH 8.0), 1 mM EDTA (pH 8.0)). By using this as a template, 3' RACE and 5' RACE were carried out.

### (c) 3' RACE

The base sequence corresponding to the N terminal section of the amino acid sequence of the royal jelly peptide was presumed. Based on this, the degenerate sense primer (37 bases of AARACNWSNATHWSNGTNAARGGNGARWSNAAYGTNG, which is hereinafter referred to as primer 1) for use in 3' RACE, shown in the sequence No. 1 was designed, and synthesized at NIHON GENE RESEARCH LABORATORIES Inc. Incidentally, in this primer, "A" represents adenine; "C", cytosine; "G", guanine; "T", thymine; and "I", inosine. Whereas, "R" represents adenine or guanine; "W", adenine or thymine; "S", cytosine or guanine; "H", adenine, cytosine, or thymine; and "Y", cytosine or thymine.

The primer 1 was diluted with a TE buffer so as to have a concentration of 100 pmol/µl to be used. For the 3' RACE reaction, with the cDNA ligated with the adaptor in the item (IV) (b) as a template, the primer 1, AP1 primer to be specifically ligated with Marathon cDNA Adaptor, and Ampli Tag Gold (trade name) (manufactured by PERKIN ELMER Co.) were used. The adaptor-ligated cDNA was added into 50 µl of a PCR buffer containing 10 nmol dNTP, 10 pmol of AP1 primer, 100 pmol primer 1, and 2.5 units of Ampli Tag Gold (trade name). Thus, by using a thermal cycler (Gene Amp PCR SYSTEM 9700, manufactured by PERKIN ELMER Co.), the PCR reaction was carried out under the following conditions. Namely, 3 cycles of (1) 95 °C, 10 minutes (heat treatment), (2) 94 °C, 1 minute (thermal denaturation), (3) 72 °C, 1 minute (annealing), and (4) 72 °C, 1 minute (extension) were carried out. Subsequently, the annealing temperature of the step (3) was reduced to 68 °C, 64 °C, 60 °C, and 56 °C, and thus, 3 cycles were carried out respectively. Then, finally, the annealing temperature was set at 52 °C, and the reaction was conducted for 25 cycles, and an extension reaction at 72 °C for 7 minutes was added thereto.

Incidentally, although not shown, agarose electrophoresis was carried out, thereby to observe the PCR products.

### (d) Subcloning of PCR products

In order to determine the base sequence of the PCR products obtained from the item (c) of the above <4>, subcloning of the PCR products was carried out according to the attached protocol by means of an Original TA Cloning Kit (manufactured by Invitrogen Co.). In summary, by using the activity of the Taq polymerase, adenine was added to the 3' ends of the PCR products. The PCR products were inserted and ligated into plasmid vectors pCR2.1 from which one thymine projects.

The PCR products were subjected to agarose gel electrophoresis which is an existing process. Then, they were visualized at a wavelength of 312 nm on a UV Transilluminator (AE-6991CX, manufactured by ATTO Co.). Thus, the objective band portions of the PCR products were cut out, and the PCR products were collected by means of SUPREC-01 (trade name) (manufactured by Takara Shuzo Co., Ltd.) which is a centrifugal tube including a filter for collecting DNA.

A part of the collected PCR products (3.5 µl) was allowed to react at 14 °C overnight in 10 µl of a solution containing 6 mM Tris-HCl (pH 7.5), 6 mM MgCl₂, 5 mM NaCl, 100 µg/ml BSA, 7 mM β-mercaptoethanol, 100 µM ATP, 2 mM DTT, 1 mM spermide, 4.0 weiss units of T4 DNA Ligase, and 50 ng of pCR 2.1 plasmid vectors. Thus, the PCR products were ligated to pCR 2.1 plasmid vectors. With the vectors, the competent cell (INV-α F' One Shot Cell) included in Original TA Cloning Kit was transformed, and then, inoculated / applied to an X-Gal and kanamycin coated LB agar plate. Then, the plate was cultured at 37 °C overnight to perform color selection.

Then, white colonies were selected, and insert check was carried out. Namely, the colonies were picked with toothpicks, and inserted in 20 µl of a PCR reaction solution containing 4 nmol dNTP, 4 pmol AP1 primer, 40 pmol primer 1, and 1 unit of Ampli Tag Gold (trade name). Thus, the colony attached on the toothpick was washed. Then, the PCR reaction was effected under the same conditions as in the item <4> (c). Thus, the phoretic pattern was observed with electrophoresis.

The colonies which had been confirmed to contain the PCR products with an expected molecular weight from the results were inoculated into a 2xYT Broth (1 % (W/V) NaCl, 1.6 % (W/V) tripton, 1 % (W/V) yeast extract), and cultured with shaking at 37 °C at 205 rpm for 8 hours.

### (e) Plasmid extraction

From the Escherichia coli cultured in the 2xYT Broth, DNA was adsorbed on magnetic beads according to the attached protocol by means of 1 MagExtractor-Plasmid-kit (manufactured by TOYOBO Co., Ltd.) to extract a plasmid.

### (f) DNA sequence

1.5 µg of the plasmid obtained by the item <4> (e), Texas-Red labeled M13(-20) reverse primer (Thermo Sequenase pre-mixed cycle sequencing kit: manufactured by Amersham Pharmacia Biotech Co.), and sterilized water were mixed. The mixture was added to a solution containing respective bases of A, C, G, and T (Thermo Sequenase fluorescent labeled cycle sequencing kit: manufactured by Amersham Pharmacia Biotech Co.). Thus, the sequence reaction was carried out in 25 cycles under the following conditions. Namely, the reaction was carried out under the conditions: (1) 95 °C, 3 minutes (heat treatment), (2) 95 °C, 1 minute (heat denaturation), (3) 50 °C, 45 seconds (annealing), (4) 72 °C, 1 minute and 30 seconds (extension), and (5) 72 °C, 1 minute (extension reaction). To the reaction solution, a 2x loading dye was added, and the mixture was concentrated by an aspirator. The resulting concentrate was loaded in an amount of 3 µl at one time onto a DNA autosequencer (SQ-5500, manufactured by Hitachi Ltd.) to analyze the base sequence.

### (g) 5' RACE

Based on the base sequence information of the royal jelly peptide obtained from 3' RACE, two types of Gene Specific antisense primers for 5' RACE were designed and produced. Namely, the two types of primers are primer 2 (sequence No. 2) designed so as to have a partially overlapping region with the sequence obtained by 3' RACE, and primer 3 (sequence No. 3) intended to acquire the base sequence of the full-length cDNA based on the base sequence on the 3' end side.

Incidentally, 5' RACE was carried out with the same process and under the same conditions as the method and conditions for the 3' RACE exemplified in the item (IV) (c). However, the reaction of the 5' RACE was carried out by using the primer 2 or the primer 3 in place of the primer 1. The obtained PCR products were subjected to subcloning and analysis of the base sequence in the same manner as with the items <4> (d) to <4> (f).

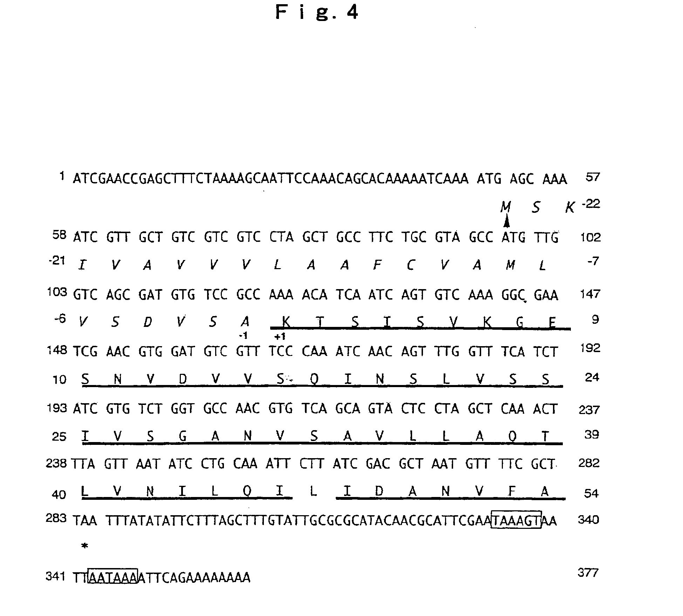

With the 3' RACE and 5' RACE shown in the items <4> (c) and <4> (g), the base sequence of the full-length cDNA of the royal jelly peptide was determined as shown in FIG. 4 (sequence No. 16 and sequence No. 17). Accordingly, the 47th amino acid which had been unidentified at Example 3 was found to be leucine. Incidentally, in the figure, the italic represent "signal sequence"; the arrow, "starting point"; the mark *, "termination codon"; the underline, "the same site as the N terminal amino acid sequence of FIG. 3"; and the box, "polyadenylation signal".

The foregoing results indicated that the royal jelly peptide of the invention of this application is apisimin (Bilikova, K., et al., FERS Letters, 528, p125 to 129, 2002).

### (h) Confirmation of base sequence with the PCR reaction using La Taq polymerase

The Ampli Taq Gold (trade name) of Taq polymerase used in the 3' RACE and the 5' RACE has no 3'→5'exonuclease activity, and hence one misreading may occur per 440 bases. For this reason, the PCR reaction using LA-Taq (manufactured by Takara Shuzo Co., Ltd.) having 3'→5'exonuclease activity was carried out to minimize the possibility of misreading. Thus, the base sequence of the royal jelly peptide of the invention of this application was confirmed (not shown).

Incidentally, the reaction was carried out in the following manner. In 50 µl of a reaction solution containing 20 nmol dNTP, 125 nmol MgCl₂, 10 pmol AP1 primer, 100 pmol primer 3, and 2.5 units of LA-Taq, the adaptor-ligated cDNA was added as a template. The mixture was subjected to 25 cycles of (1) 94 °C, 30 seconds (heat denaturation), (2) 50 °C, 30 seconds (annealing), (3) 72 °C, 1 minute and 30 seconds (extension), and (5) 72 °C, 1 minute (extension reaction). Thus, the base sequence was confirmed by means of a DNA sequencer in the same manner as with the item <4> (f).

### (i) Analysis by computer

For homology search, BLAST was used. For the estimation of the position of the starting codon, ORF Finder was used.

### Example 5: Analysis of royal jelly peptide (apisimin)

### <1> Southern blotting process

By using the genome DNA of Western honeybees, the analysis of the gene of the royal jelly peptide was carried out by employing the Southern blotting process which is a known process.

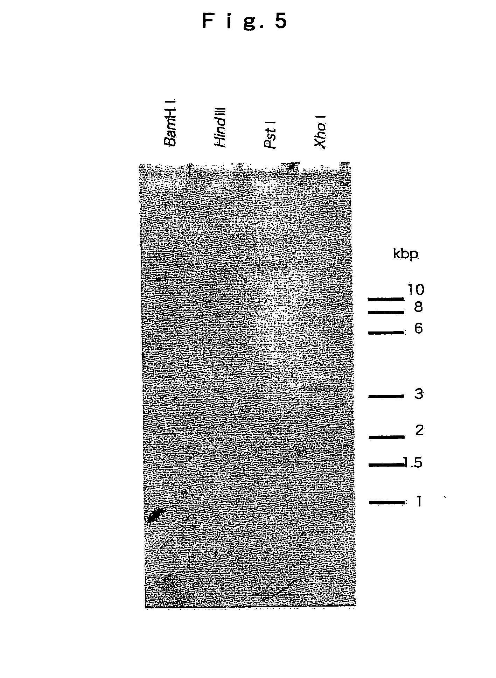

As a result, as shown in FIG. 5, the gene of the royal jelly peptide (apisimin) shows the feature of the class II gene having two introns, and can be judged as being 1 copy per haploid genome.

Incidentally, in the figure, the lane "BamH I" represents a restriction enzyme BamH I-treated sample; the lane "Hind III", a restriction Hind III-treated sample; "Pst I", a restriction enzyme Pst I-treated sample; and "Xho I", a restriction enzyme Xho I-treated sample.

### <2> Northern blotting process

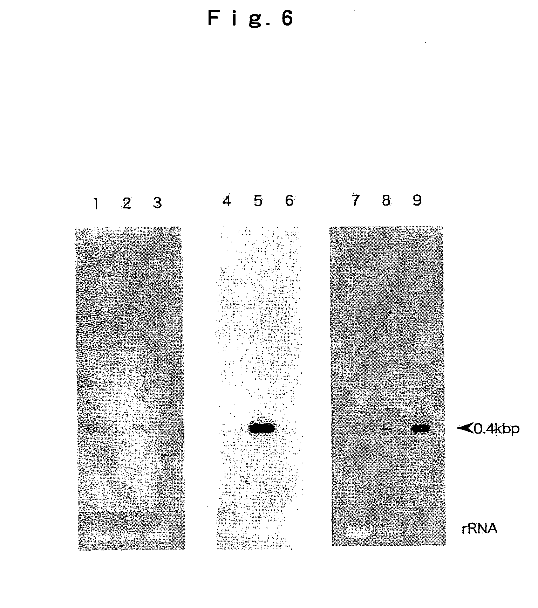

Whereas, with the Northern blotting process, the relationship between the age in day (stage) and the organ of Western honeybees and the gene expression of the royal jelly peptide was analyzed. As a result, as shown in FIG. 6, the strong expression was also observed in the hypopharyngeal gland of the working bee which had undergone differentiation from the nurse bee to the foraging bee.

Incidentally, the lanes 1 to 6 represent 10 µg of total RNA extraction from working bees (nurse bees) 7 days after eclosion, and represent the heads, the thoraces, the abdomens, the brains, the hypopharyngeal glands, and the mandibular glands, respectively. Whereas, the lane 7 represents 10 µg of total RNA extracted from the hypopharyngeal glands 0 day after eclosion; the lane 8, from the hypopharyngeal glands 7 days after eclosion; and the lane 9, from the hypopharyngeal glands of working bees (foraging bees) 30 days after eclosion.

### Example 6: Expression system of royal jelly peptide (apisimin)

According to a known method (e.g., Sambrook, J., et al., Molecular Cloning Cold Spring Harbor Laboratory Press, 1989), the gene of the royal jelly peptide of this application was transferred into Escherichia coil to construct a large scale expression system. Thus, expression was carried out in the form of a recombinant royal jelly peptide.

### <1> Construction of royal jelly peptide gene

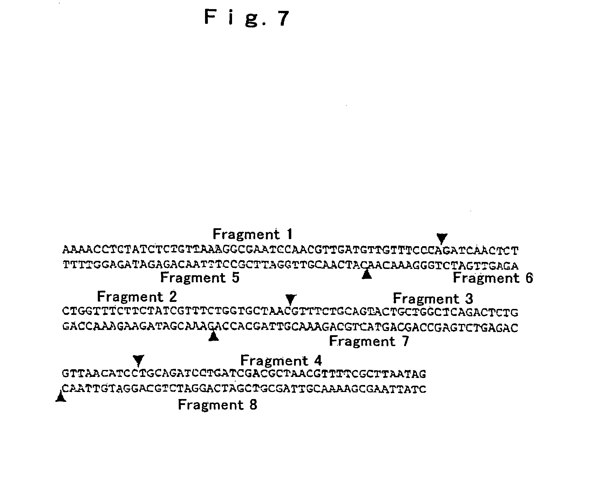

The royal jelly peptide gene used in this example was obtained in the following manner. The base sequence is designed with reference to the high usage codons in Escherichia coil. Based on this, the oligonucleotides as exemplified in FIG. 7 (fragments 1 to 8) were produced, followed by a PCR reaction. The produced oligonucleotides include respective 4 types (a total of 8 types) on the sense side and on the antisense side, which are designed so that the portions to be annealed for 10 bases overlap one another.

Incidentally, the fragment 1 is indicated as sequence No. 4; the fragment 2, sequence No. 5; the fragment 3, sequence No. 6; the fragment 4, sequence No. 7; the fragment 5, sequence No. 8; the fragment 6, sequence No. 9; the fragment 7, sequence No. 10; and the fragment 8, sequence No. 11.

As a specific process, the fragments 2 to 7 (50 pmol each) and a reaction solution (50 mM Tris-HCl (pH 8.0), 10 mM MgCl₂, 5 mM DTT, 0.5 mM ATP) containing T4 polynucleotide Kinase (10 U/20 µl) were allowed to react with each other at 37 °C for 30 minutes, thereby to effect the phosphorylation of the 5' end. The sample after the reaction was extracted with phenol / chloroform / isoamyl alcohol (PCI), and collected with ethanol precipitation. Then, the respective fragments were mixed, and an annealing reaction was carried out. For annealing, a reaction solution (10 mM Tris-HCl (pH 7.5), 10 mM MgCl₂, 1 mM DTT, 50 mM NaCl) containing the fragments 1 and 8 (10 pmol each) and the fragments 2 to 7 (10 pmol each) subjected to the phosphorylation was mixed. The sample was set in a thermal cycler to effect the reaction. For the condition program of this reaction, the program was set such that heat denaturation was carried out at 95 °C for 5 minutes, and then, the temperature was reduced to 25 °C for 90 minutes. Thus, the annealing reaction was carried out. A double stranded DNA was produced by this reaction, and by using this as a template DNA, the PCR reaction was carried out. The PCR reaction was carried out in a reaction solution (10 mM Tris-HCl (pH 8.3), 50 mM KCI, 1.5 mM MgCl₂, 0.001 % gelatin, 200 µm dNTP, 1.25 U/50 µl Ampli Taq Gold) containing the template DNA (1 µl), and a sense primer and an antisense primer (0.4 µl each). Incidentally, as the sense primer, a LIC-forward primer (sequence No. 12) was used, and for the antisense primer, a LIC-reverse primer (sequence No. 13) was used. To each of the primers, the sequence specific to the expression vector is added.

The PCR products obtained by the PCR reaction was subjected to subcloning by means of a TA Cloning Kit. It was verified by a DNA sequencer that no missense occurred in the base sequence of the obtained plasmid (not shown).

### <2> Production of expression vector

The expression vector in this example was produced by using pET32 XA/LIC (manufactured by Novagen Co.) of Ligation-independent cloning (LIC) Vector for expressing the fusion protein of thioredoxin containing S-Tag and His-Tag according to the attached protocol. The PCR products obtained in the item (I) were separated by agarose gel electrophoresis, and purified with Suprec-01 (trade name), and collected with ethanol precipitation. For the formation of the LIC sites of the PCR products, the region from the 3' end to immediately adjacent to the guanine base (G) of the PCR products was cut using the 3'→5'exonuclease activity of T4 DNA Polymerase. Namely, a reaction solution (50 mM Tris-HCl (pH 7.5), 10 mM MgCl₂, 10 mM β-ME, 5 mM DTT, 2.5 mM dGTP) containing the PCR products (0.2 pmol) and T4 DNA Polymerase (1 U/20 µl) was mixed, and the annealing reaction was carried out at 22 °C for 1 hour. 1 µl of the reaction solution was transformed into a competent cell (E. coli BL21 (DE3), manufactured by Novagen Co.) with a known method. Whether the objective gene coding for the royal jelly peptide was present or not in the transformed E. coli strain was verified by the colony PCR.

### <3> Expression and purification of recombinant protein

The E. coli strain in which the royal jelly peptide gene had been transferred obtained by the item <2> was inoculated in 3 ml of an LB Broth, and precultured at 37 °C overnight. After preculturing, 2 ml of the culture solution was added to 200 ml of the same culture solution LB Broth as the culture solution used for preculturing, and culturing was carried out at 37 °C for 2 hours. Then, IPTG with a final concentration of 1 mM was added, and culturing was further carried out at 37 °C for 3 hours. Thus, the gene expression was induced. Then, cell bodies were extracted in PBS containing PMSF (final concentration 0.625 µg/ml) by means of a Polytron type homogenizer (ultra-turrax T8, IKA). Then, centrifugation was carried out at 25000 x g for 20 minutes to collect the supernatant, resulting in extracted fractions. Purification of fusion proteins (i.e., royal jelly peptide included) from the extracted fractions was carried out by using His-Tag attached to the fusion proteins. Specifically, purification was carried out by metal chelate affinity chromatography by a resin in which Ni ions to bind to His-Tag were immobilized. For the affinity resin, Ni-NTA Agarose (manufactured by Qiagen Co.) was used, and purification was carried out by a batch process. To Ni-NTA Agarose equilibrated with PBS, the extracted fractions were added, and gently mixed under low temperature conditions to be bound to the resin. Then, centrifugation was carried out at 700 x g for 3 minutes to remove the supernatant. The resin was washed with PBS. Further, in order to elute proteins nonspecificly binding to the resin, PBS containing 0.05 M Imidazole was added to wash the resin. Subsequently, PBS containing 0.5 M Imidazole was added thereto, and gently mixed under low temperature conditions to elute the objective fusion proteins. Then, the eluted fusion proteins were subjected to centrifugation at 700 x g for 3 minutes to collect the supernatant. The supernatant was substituted with a buffer for enzyme treatment (50 mM Tris-HCl (pH 8.0), 100 mM NaCl, 1 mM CaCl₂) with ultrafiltration (Ultrafree-4 centrifugal filter unit, MWCO 10000, manufactured by Millipore Co.). Then, Factor Xa (Protein Engineering Technology Aps) was added in an amount of 5 µg per 1 mg of fusion proteins. Thus, a digestion reaction was carried out at 37 °C for 24 hours.

The reaction solution digested as described above was purified with a high performance liquid chromatography (HPLC) system connected with a column for a reversed phase chromatography (RESOURCR RPC 3 ml, manufactured by Pharmacia Biotech Co.). The sample was injected into the column equilibrated with 0.1 % TFA, and eluted at the 0.1 % TFA-containing acetonitrile concentration gradient at a flow rate of 1 ml/min. For the detection, the absorbance at a wavelength of 215 nm was measured by means of an uitravioiet spectrophotometer detector, and the changes in absorbance were recorded on a recorder.

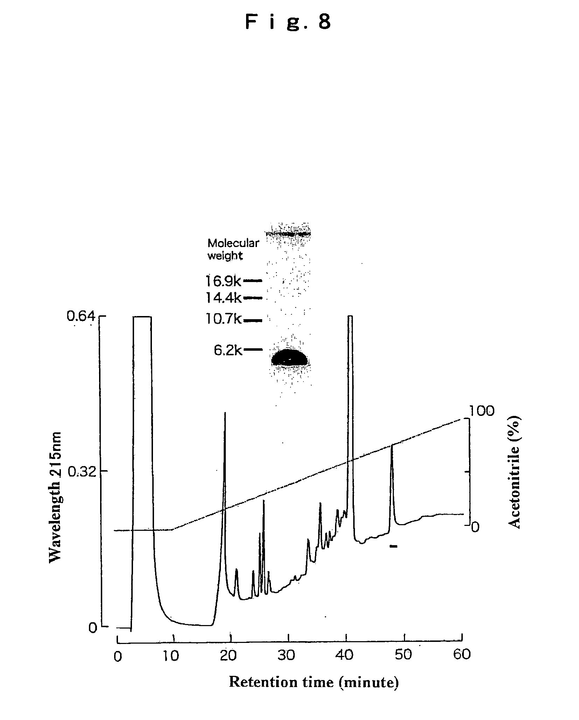

The results are as shown in FIG. 8. Whereas, the peaks on the horizontal bar in the figure were analyzed with tricin SDS-PAGE, and it was verified that the objective royal jelly peptide was isolated.

### Example 7: Functional analysis of royal jelly peptide (apisimin)

### <1> Effect of inducing differentiation to queen bees

Functional analysis was carried out by using the royal jelly peptide obtained in Example 6. Specifically, honeybees were bred according to the existing honeybee artificial breeding method. In this process, 500 ng of the royal jelly peptide was added in the artificial diet to be administered per honeybee. On the other hand, for the control group, bovine serum albumin was used in place of the royal jelly peptide.

The results are as shown in Table 1. The growth rate from larvae to imagines is about 20 %. However, intermediates can be allowed to emerge out of the larvae to which the royal jelly peptide had been administered. From this, it can be considered that the royal jelly peptide of the invention of this invention is a factor having an auxiliary function in inducing differentiation to queen bees.

**Table 1**

| | Number of first instar larvae | Number of last instar larvae | Number of pupae | Number of imagines | Queen | Caste intermediates | Working bee |
|---|---|---|---|---|---|---|---|
| Control group (Bovine serum albumin) | 24 | 14 | 9 | 2 | 0 | 0 | 2 |
| Recombinant peptide | 24 | 14 | 8 | 4 | 0 | 1 | 3 |

### <2> Immunity activation test

### (a) Laboratory animal and medium composition

For the laboratory animal, ICR type mice (female, Nippon SLC) were used.

Whereas, for the medium, an RPMI medium (manufactured by Nissui Pharmaceutical Co., Ltd.) containing 10 % fetal calf serum (FCS), 2 mM L-glutamine, 50 µM 2-mercaptoethanol, 100 Units/ml penicillin, and 100 µg/ml streptomycin was used. Thus, culturing was carried out in 5 % CO₂ at 37 °C under wet conditions.

### (b) Control of cell suspension

Control of a cell suspension was carried out by the following steps.

Namely, the mice anesthetized with diethyl ether were subjected to cervical vertebrae dislocation, and fixed. Then, by using scissors for dissection and tweezers, the spleen was excised, and transferred to a dish containing therein 10 ml of PBS (-) (137 mM NaCl, 2.7 mM KCI, 2 mM Na₂HPO₄·12H₂O, 1.5 mM KH₂PO₄). In the dish, the spleen was homogenized. This was filtrated into a 50-ml tube (manufactured by FALCON Co.) by using a metal mesh, and controlled as a cell solution. This was subjected to centrifugation at 1100 rpm for 10 minutes. 5 ml of a Tris ammonium chloride solution (0.14 M ammonium chloride, 1.7 mM Tris-HCl (pH 7.65) was added thereto, and incubation was carried out for 5 minutes. This operation was repeated twice to remove red blood cells.

Subsequently, 30 ml of PBS(-) for washing was added thereto, and centrifugation was repeated twice at 1100 rpm for 10 minutes to remove the supernatant. Then, 40 ml of the RPMI medium of the item (a) was added thereto, and the mixture was transferred to a flask. Thus, incubation was carried out at 37 °C for 2 hours in a CO₂ incubator (manufactured by WAKENYAKU Co., Ltd.), and the supernatant in the flask was transferred to a new tube. The resulting one was taken as a lymph cell suspension.

The lymph cell suspension controlled was dyed with a 0.4 % trypan blue solution, and the number of cells was counted by means of a Burker-Turk hemocytomer. The one controlled in the medium so that the number of cells becomes 1 × 10⁶ cells/ml was used for the immunity activation test.

### (c) Immunity activation test

The lymph cell suspension controlled in the item <b> was added in an amount of 100 µl per well into a 96-well microplate (manufactured by IWAKI Co.). The cell solution was dissolved in dimethyl sulfoxide (DMSO) so that the final concentration of the lymph cell suspension was 1 %, and added in an amount of 11 µl in each well. Simultaneously therewith, alamar blue (trade name) (manufactured by WAKO PURE CHEMICAL Industries Ltd.) capable of measuring the reducing power by the metabolic activity of the immunocyte (in the case of this example, lymphocyte cell) was added in an amount of 10 µl in each well. Thus, the changes in absorbance were measured by means of a microplate reader (manufactured by Nalge Nunc International Co.) set at a wavelength of 590 to 620 nm every 12 hours for 4 days to analyze the effects of each fraction and isolated compound on the lymphocytes.

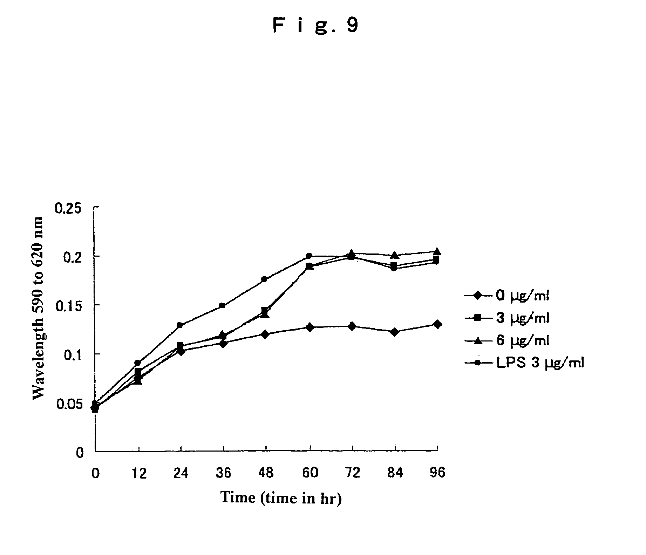

The results are as shown in FIG. 9. By comparing the case where the royal jelly peptide was not added with the case where it was added, it could have been shown that the effects of the royal jelly peptide of the invention of this application are remarkably exhibited.

In the figure, a black rhomboid represents an additive-free control; a black circle, the one containing lipopolysaccharide (LPS) added in an amount of 3 µg/ml therein of a positive control; a black box, the one containing a recombinant royal jelly peptide added in an amount of 3 µg/ml therein; and a black delta, the one containing a recombinant royal jelly peptide added in an amount of 6 µg/ml therein.

Example 8: Repression of induction of differentiation to queen bees using anti royal jelly peptide antibody

### <1> Production of anti royal jelly peptide antibody

A peptide "CSIVSGANVSAVL" which can be an antigen was synthesized using the 24th to 35th amino acid sequences based on the amino acid sequences (sequence No 14 to sequence No. 17) of the royal jelly peptide shown in FIGS. 3 and 4. In order to produce the antibody with a common process by using this synthetic peptide as an antigen, this was administered in an amount of 1 mg per dose to two rabbits (breed: UW Clean) to carry out an immunosensitization experiment. In from 5 weeks to 7 weeks from the start of the immunosensitization experiment, collection of blood of the samples was performed which carrying out booster. While examining the antibody titer with ELISA, the whole blood was finally collected, and the serum was subjected to the subsequent experiment.

### <2> Affinity purification process using antigen peptide

20 ml of a 40 % ammonium sulfate precipitate of the blood serum was dialyzed with 20 mM phosphoric acid and 150 mM NaCl Buffer (pH 7.4), and filtrated through a 0.45-µm filter, resulting in a sample for purification.

About 10 mg of antigen peptide was bound to about 2 ml of HNS-activated Sepharose 4FF carrier to prepare an Affinity column. An equilibrating buffer (20 mM phosphoric acid, 150 mM NaCl Buffer (pH 7.4)) was flushed, and a sample was applied and circulated with the equilibrating buffer in the column for 2 hours, and then, the equilibrating buffer was flushed until return to the base line. The antibody in the blood serum was eluted with an elution buffer (100 mM citric acid-NaOH, (pH 3.0)).

The elution peak was collected at an absorbance of 280 nm, and dialyzed with PBS(-), and filtrated through a 0.45-µm filter. Then, 6 ml of a purified product could be obtained. The total protein amount was 355 µg.

### <3> Western blotting using purified antibody

The hypopharyngeal glands were excised from inside working bees, and the protein sample was separated with Trisine SDS-PAGE. The gel after separation was transferred to a PVDF film with a semi-dry system plotting process. The PVDF film after transfer was immersed in TBS-T containing 5 % skim milk as a blocking agent overnight to carry out a blocking treatment. Then, after washing with TBS-T, the purified antibody obtained in the item <2> was 100 times diluted with an antibody diluent (1 % gelatin / TBS-T). As the first antibody reaction, the PVDF film was immersed in the antibody solution, and the reaction was allowed to proceed at room temperature for 1 hour. Then, washing was carried out with TBS-T again. As the second antibody reaction, the reaction with the second antibody (Horseradish peroxidase-labeled anti-rabbit IgG goat IgG) diluted to 2000 times with an antibody eluent was allowed to proceed at room temperature for 1 hour. For the detection, chemiluminescence was caused by using ECL (trade name), and signals were exposed to an X-ray film.

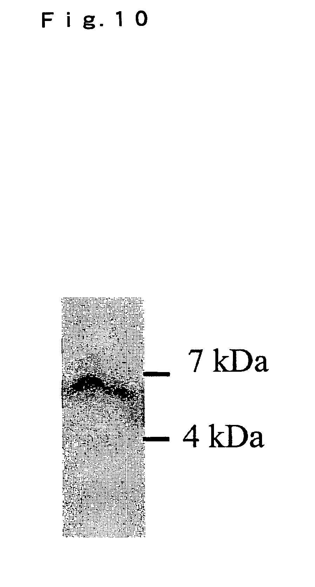

The results are as shown in FIG. 10. As shown in FIG. 10, a signal band in the protein sample in the hypopharyngeal glands from the inside working bees and the produced antibody (anti royal jelly peptide antibody) was observed in the vicinity of a molecular weight of 5500. Namely, it could have been shown that the organ for synthesizing the royal jelly peptide is the hypopharyngeal gland. Further, this result could revealed that the produced antibody is reactive with the royal jelly peptide.

### <4> Royal jelly peptide purification antibody for repressing the effect of inducing differentiation to queen bees

By using the purification antibody obtained in the items <1> to <3>, honeybees were bred according to an existing honeybee artificial breeding method. Herein, the royal jelly extraction fraction group (the one obtained by controlling the extraction fraction with a molecular weight of 100 to 10000 from the royal jelly to 50 mg/7ml) in the artificial diet was taken was a positive control. For the control group, bovine serum albumin (50 ml/7 ml) was used. For the royal jelly peptide antibody group, the one obtained by controlling 50 mg of the extraction fraction with a molecular weight of 100 to 10000 from the royal jelly taken as the positive control to 7ml, and adding 20 µg of antibody thereto was used. The results are shown in Table 2.

**Table 2**

| | Number of first instar larvae | Number of last instar larvae | Number of imagines | Queen bee | Intermediate | Working bee |
|---|---|---|---|---|---|---|
| Control group (bovine serum albumin 50 mg/7 ml) | 48 | 34 | 6 | 0 | 3 | 3 |
| Royal jelly extraction fraction group (peptide fraction with a molecular weight of 100 to 10,000 50 mg/7ml) | 24 | 20 | 4 | 1 | 3 | 0 |
| Royal jelly peptide antibody group (the extraction fraction 50 mg/7 ml + antibody 20 µg/7ml) | 24 | 24 | 7 | 0 | 6 | 1 |

As shown in Table 2, for the control group, the growth rate from larvae to imagines was about 13 %; for the royal jelly extraction fraction group, about 17 %; and for the royal jelly peptide antibody group, 29 %. Out of the respective ones grown into imagines, for the control, each emergence rate of intermediates and working bees was 50 %. For the royal jelly extraction fraction group of a positive control, 25 % became queen bees, 75 % became intermediates, and working bees did not emerge at all. For the royal jelly peptide antibody group, the emergence of queen bees was not observed, the intermediate accounted for 86 %, and the emergence rate of working bees was also 14 %.

The results indicate the following fact. By adding a royal jelly peptide antibody with such a low concentration as 0.04 % (20 µg ÷ 50 mg) of the royal jelly extraction fraction into the artificial diet, the effect of repressing the induction to queen bees are produced. Therefore, the results could reconfirm that the royal jelly peptide is a factor having an auxiliary function in inducing differentiation to queen bees.

### Industrial Applicability

As described in details up to this point, the invention of this application provides a royal jelly peptide which has a function as a cofactor for induction to queen bees, and can implement the improvement of the biological defense system of an animal (immunity activating effect), and a composition containing the same.

## Claims

1. A royal jelly peptide **characterized by** having at least any function activity of a function as a queen bee differentiation inducing cofactor, and a function as an animal immunity activating factor.

2. An expression vector including an expression cassette having a gene sequence coding for the royal jelly peptide according to claim 1.

3. A transgenic cell transformed by the expression vector according to claim 2.

4. A royal jelly peptide produced by the transgenic cell according to claim 3.

5. A composition containing the royal jelly peptide according to claim 1 or 4 as an effective component, **characterized by** having at least any function of a function of improving the auxiliary effect of inducing differentiation of honeybees to queen bees, and a function of improving the animal immunity activating effect.

6. A method for inducing honeybees to queen bees **characterized by** administering the composition according to claim 5 to honeybee larvae, thereby promoting the growth of the larvae, and inducing differentiation to queen bees.

7. A method for activating the animal immunity, **characterized by** administering the composition according to claim 5 to an animal, and thereby improving the animal immunity activating effect.

8. The transgenic organisms being a non-human organism obtained by ontogenesis of any of an early embryo, a fertilized egg, and an embryonic stem cell of a non-human organism into which a gene sequence coding for the royal jelly peptide according to claim 1 has been transferred, and a progeny organism thereof, **characterized by** having the gene in a somatic cell chromosome, and expressing the royal jelly peptide.

9. The non-human organism according to claim 8, **characterized in that** the non-human organism is any of a plant or an animal.

10. The non-human organism according to claim 9, **characterized in that** the animal is an insecta.

11. The non-human organism according to claim 10, **characterized in that** the insecta is a silkworm.


SEQUENCE LISTING

<110> Japan Science and Technology Agency

<120> Royal Jelly Peptide

<130> 04F039PCT

<150> JP 2003-338665
<151> 2003-09-29

<160> 17

<170> PatentIn version 3.1

<210> 1
<211> 37
<212> DNA
<213> Artificial Sequence

<220>
<223> Primer 1

<220>
<221> misc_feature
<222> (1)..(37)
<223> i

<400> 1
aaracnwsna thwsngtnaa rggngarwsn aaygtng 37

<210> 2
<211> 29
<212> DNA
<213> Artificial Sequence

<220>
<223> Primer 2

<400> 2
cgttggcacc agacacgata gatgaaacc 29

<210> 3
<211> 29
<212> DNA
<213> Artificial Sequence

<220>
<223> Primer 3

<400> 3
tttctgaatt ttattaatta ctttattcg 29

<210> 4
<211> 50
<212> DNA
<213> Artificial Sequence

<220>
<223> Fragment 1

<400> 4
aaaacctcta tctctgttaa aggcgaatcc aacgttgatg ttgtttccca 50

<210> 5
<211> 40
<212> DNA
<213> Artificial Sequence

<220>
<223> Fragment 2

<400> 5
gatcaactct ctggtttctt ctatcgtttc tggtgctaac 40

<210> 6
<211> 40
<212> DNA

<213> Artificial Sequence

<220>
<223> Fragment 3

<400> 6
gtttctgcag tactgctggc tcagactctg gttaacatcc 40

<210> 7
<211> 38
<212> DNA
<213> Artificial Sequence

<220>
<223> Fragment 4

<400> 7
tgcagatcct gatcgacgct aacgttttcg cttaatag 38

<210> 8
<211> 40
<212> DNA
<213> Artificial Sequence

<220>
<223> Fragment 5

<400> 8
ttttggagat agagacaatt tccgcttagg ttgcaactac                                40

<210> 9
<211> 40
<212> DNA
<213> Artificial Sequence

<220>
<223> Fragment 6

<400> 9
aacaaagggt ctagttgaga gaccaaagaa gatagcaaag                                40

<210> 10
<211> 40
<212> DNA
<213> Artificial Sequence

<220>
<223> Fragment 7

<400> 10
accacgattg caaagacgtc atgacgaccg agtctgagac                                40

<210> 11

<211> 48

<212> DNA

<213> Artificial Sequence

<220>

<223> Fragment 8

<400> 11

caattgtagg acgtctagga ctagctgcga ttgcaaaagc gaattatc 48

<210> 12

<211> 31

<212> DNA

<213> Artificial Sequence

<220>

<223> LIC Forward

<400> 12

ggtattgagg gtcgcaaaac ctctatctct g 31

<210> 13

<211> 33

<212> DNA

<213>  Artificial Sequence

<220>
<223>  LIC Reverse

<400>  13
agaggagagt tagagcccta ttaagcgaaa acg                                    33

<210>  14
<211>  162
<212>  DNA
<213>  bee

<220>
<221>  misc_feature
<222>  (1)..(162)
<223>  unknown

<220>
<221>  CDS
<222>  (1)..(162)
<223>

<400>  14
aaa aca tca atc agt gtc aaa ggc gaa tcg aac gtg gat gtc gtt tcc        48
Lys Thr Ser Ile Ser Val Lys Gly Glu Ser Asn Val Asp Val Val Ser

1                5                   10                  15

caa atc aac agt ttg gtt tca tct atc gtg tct ggt gcc aac gtg tca        96
Gln Ile Asn Ser Leu Val Ser Ser Ile Val Ser Gly Ala Asn Val Ser
            20                  25                  30

gca gta ctc cta gct caa act tta gtt aat atc ctg caa att nnn atc        144
Ala Val Leu Leu Ala Gln Thr Leu Val Asn Ile Leu Gln Ile Xaa Ile
        35                  40                  45

gac gct aat gtt ttc gct                                                162
Asp Ala Asn Val Phe Ala
    50

<210>  15
<211>  54
<212>  PRT
<213>  bee

<220>
<221>  misc_feature
<222>  (47)..(47)
<223>  The 'Xaa' at location 47 stands for Lys, Asn, Arg, Ser, Thr, Ile,
        Met, Xaa, Glu, Asp, Gly, Ala, Val, Gln, His, Pro, Leu, Tyr, Trp,
        Cys, or Phe.

<400>  15

Lys Thr Ser Ile Ser Val Lys Gly Glu Ser Asn Val Asp Val Val Ser
1               5                   10                  15

Gln Ile Asn Ser Leu Val Ser Ser Ile Val Ser Gly Ala Asn Val Ser
            20                  25                  30

Ala Val Leu Leu Ala Gln Thr Leu Val Asn Ile Leu Gln Ile Xaa Ile
        35                  40                  45

Asp Ala Asn Val Phe Ala
    50

<210>  16
<211>  162
<212>  DNA
<213>  bee

<220>
<221>  CDS
<222>  (1).. (162)
<223>

<400>  16

aaa aca tca atc agt gtc aaa ggc gaa tcg aac gtg gat gtc gtt tcc 48
Lys Thr Ser Ile Ser Val Lys Gly Glu Ser Asn Val Asp Val Val Ser
1               5                   10                  15

caa atc aac agt ttg gtt tca tct atc gtg tct ggt gcc aac gtg tca 96
Gln Ile Asn Ser Leu Val Ser Ser Ile Val Ser Gly Ala Asn Val Ser
            20                  25                  30

gca gta ctc cta gct caa act tta gtt aat atc ctg caa att ctt atc 144
Ala Val Leu Leu Ala Gln Thr Leu Val Asn Ile Leu Gln Ile Leu Ile
        35                  40                  45

gac gct aat gtt ttc gct 162
Asp Ala Asn Val Phe Ala
    50

<210> 17
<211> 54
<212> PRT
<213> bee

<400> 17

Lys Thr Ser Ile Ser Val Lys Gly Glu Ser Asn Val Asp Val Val Ser
1               5                   10                  15

Gln Ile Asn Ser Leu Val Ser Ser Ile Val Ser Gly Ala Asn Val Ser
            20                  25                  30
Ala Val Leu Leu Ala Gln Thr Leu Val Asn Ile Leu Gln Ile Leu Ile
        35                  40                  45
Asp Ala Asn Val Phe Ala
    50